# EUROPEAN PATENT APPLICATION

(11) **EP 2 883 952 A1**
(43) Date of publication of application: **17.06.2015**
(21) Application number: 13827242.2
(22) Date of filing: 09.08.2013
(51) Int. Cl.: C12N 1/12

(54) **CULTURE APPARATUS FOR MICROALGAE AND CULTURE METHOD FOR MICROALGAE**

(30) Priority: 10.08.2012 JP 2012178762; 12.12.2012 JP 2012271509
(71) Applicant: Kobelco Eco-Solutions Co., Ltd, Kobe-shi, Hyogo 651-0072 (JP)
(72) Inventor: AKASHI, Akira, Kobe-shi Hyogo 651-2241 (JP); TAKEZAKI, Jun, Kobe-shi Hyogo 651-2241 (JP); HAMADA, Takeshi, Kobe-shi Hyogo 651-2241 (JP); TERASAWA, Kiyoshi, Kobe-shi Hyogo 651-2241 (JP); TAKAHASHI, Madoka, Kobe-shi Hyogo 651-2241 (JP); OHIRAKI, Kenji, Kobe-shi Hyogo 651-2241 (JP)
(74) Representative: Stolmár & Partner Patentanwälte PartG mbB
(86) International application number: PCT/JP2013/071681
(87) International publication number: WO 2014/025020

(57) **Abstract**

Provided is a method for culturing microalgae in a liquid at least containing a brewed beverage. Further provided is a method for using microalgae that enables efficient use of organic compounds stored by the microalgae cultured by the aforementioned culture method within their cells.

## Description

### CROSS-REFERENCE TO RELATED APPLICATIONS

This application claims priority to Japanese Patent Application Nos. 2012-178762 and 2012-271509, the disclosures of which are incorporated herein by reference in their entirety.

### FIELD

The present invention relates to a method for culturing microalgae and a method for using microalgae.

### BACKGROUND

Conventionally, various methods for culturing microalgae have been known. Examples thereof include a method in which microalgae are cultured in a liquid containing nitrogen.

In such a method for culturing microalgae of this type, it is possible to store valuables such as lipids, proteins, and vitamins, which serve as raw materials for fuels, feeds, health foods, or the like, within the cells of microalgae by growing the microalgae. Then, the stored valuables can be used for fuels, foods, or the like.

As a method for culturing microalgae of this type, a method in which microalgae are cultured in a liquid containing treated sewage water generated by treating sewage such as household effluent is known (Patent Literature 1).

According to this method for culturing microalgae, the treated sewage water contains nitrogen, and therefore it is possible to grow microalgae. Further, the valuables such as hydrocarbons stored in the microalgae can be used. Furthermore, it is possible to improve the quality of the treated sewage water used for culture.

However, this method for culturing microalgae has a problem that the microalgae cannot necessarily grow efficiently. If the microalgae do not grow efficiently, the stored amount of valuables within the cells is not necessarily sufficient, and the amount of valuables that can be used is comparatively reduced.

### CITATION LIST

### Patent Literature

Patent Literature 1: JP H05-301097 A

### SUMMARY

### Technical Problem

In view of the aforementioned problem, it is an object of the present invention to provide a method for culturing microalgae, which enables efficient growth of microalgae. It is another object of the present invention to provide a method for using microalgae, which enables efficient use of organic compounds stored within the cells of the microalgae that have been cultured by the aforementioned culture method.

### Solution to Problem

In order to solve the aforementioned problem, the method for culturing microalgae according to the present invention is characterized by culturing microalgae in a liquid at least containing a brewed beverage.

According to an aspect, the method for culturing microalgae according to the present invention can employ an embodiment in which the microalgae are cultured in a liquid containing the brewed beverage and at least one selected from the group consisting of sweet wort, sake lees, shochu lees, yeast, yeast autolysate, and blackstrap molasses.

According to another aspect, the method for culturing microalgae according to the present invention can employ an embodiment in which the brewed beverage is at least one selected from the group consisting of beer, sake, and wine.

According to another aspect, the method for culturing microalgae according to the present invention can employ an embodiment in which the microalgae are cultured in the liquid containing oxygen while performing photosynthesis by irradiation with light.

According to another aspect, the method for culturing microalgae according to the present invention can employ an embodiment in which the microalgae are cultured in facilities for producing beer as the brewed beverage and in a liquid containing beer generated in the facilities.

According to another aspect, the method for culturing microalgae according to the present invention can employ an embodiment in which the microalgae are cultured in facilities for producing beer as the brewed beverage and in a liquid containing beer generated in the facilities and at least one of sweet wort, yeast, and yeast autolysate that are generated in the facilities.

According to another aspect, the method for culturing microalgae according to the present invention can employ an embodiment in which the microalgae are cultured in facilities for producing sake as the brewed beverage and in a liquid containing sake generated in the facilities.

According to another aspect, the method for culturing microalgae according to the present invention can employ an embodiment in which the microalgae are cultured in facilities for producing sake as the brewed beverage and in a liquid containing sake generated in the facilities and at least one of sake lees, yeast, and yeast autolysate that are generated in the facilities.

A method for using microalgae according to the present invention is characterized by using the microalgae obtained by the method for culturing microalgae at least as fuels in the facilities in which the culture method has been implemented.

### BRIEF DESCRIPTION OF DRAWINGS

Fig. 1 is a schematic view showing the outline of devices used in a method for culturing microalgae.
Fig. 2 is a schematic view showing the outline of devices used in the method for culturing microalgae and the outline of other devices.

### DESCRIPTION OF EMBODIMENTS

Hereinafter, an embodiment of a method for culturing microalgae according to the present invention is described in detail.

In the method for culturing microalgae of this embodiment, the microalgae are cultured in a liquid at least containing a brewed beverage.

That is, in the method for culturing microalgae, the microalgae are cultured in a liquid at least containing a brewed beverage containing nutrients that promote the growth of the microalgae.

According to the method for culturing microalgae of this embodiment, the microalgae are cultured in a liquid at least containing a brewed beverage, and therefore the microalgae can be grown efficiently.

In the method for culturing microalgae of this embodiment, it is preferable that the microalgae be cultured in a liquid containing a brewed beverage, water, and at least one selected from the group consisting of sweet wort, sake lees, shochu lees, yeast, yeast autolysate, and blackstrap molasses.

That is, in the method for culturing microalgae, it is preferable that a brewed beverage and at least one selected from the group consisting of sweet wort, sake lees, shochu lees, yeast, yeast autolysate, and blackstrap molasses be employed as materials containing nutrients that promote the growth of the microalgae, and the microalgae be cultured in a liquid containing these materials and water.

Examples of the nutrients include inorganic nutrients and organic nutrients.

Examples of the inorganic nutrients include nitrogen-containing inorganic compounds and phosphorus-containing inorganic compounds. Further, examples of the inorganic nutrients include potassium ions, iron ions, manganese ions, cobalt ions, zinc ions, copper ions, molybdenum ions, and nickel ions.

On the other hand, examples of the organic nutrients include monosaccharides such as glucose, ethanol, and vitamins.

The microalgae are organisms inhabiting in water while floating therein. Further, the microalgae are generally unicellular, being different from kelp or brown seaweed, and are algae having a very small size of about several micrometers to several tens of micrometers.

Examples of the microalgae include photoautotrophic microalgae that grow by photosynthesis and heterotrophic microalgae that grow using organic nutrients such as glucose as a nutrient source.

Examples of the photoautotrophic microalgae include organisms capable of photosynthesis and using organic nutrients as a nutrient source, like organisms belonging to the genus *Euglena,* which will be described below.

The microalgae are preferably at least one selected from the group consisting of organisms belonging to the genus *Euglena,* organisms belonging to the genus *Chlorella,* organisms belonging to the genus *Aurantiochytrium,* organisms belonging to the genus *Auxenochlorella,* organisms belonging to the genus *Botryococcus,* organisms belonging to the genus *Nannochloris,* organisms belonging to the genus *Nannochloropsis,* organisms belonging to the genus *Neochloris,* organisms belonging to the genus *Pseudochoricystis,* organisms belonging to the genus *Scenedesmus,* and organisms belonging to the genus *Schizochytorium.*

The photoautotrophic microalgae are preferably at least one selected from the group consisting of organisms belonging to the genus *Euglena,* organisms belonging to the genus *Chlorella,* organisms belonging to the genus *Auxenochlorella,* organisms belonging to the genus *Botryococcus,* organisms belonging to the genus *Nannochloris,* organisms belonging to the genus *Nannochloropsis,* organisms belonging to the genus *Neochloris,* organisms belonging to the genus *Pseudochoricystis,* and organisms belonging to the genus *Scenedesmus.*

The heterotrophic microalgae are preferably organisms belonging to the genus *Aurantiochytrium* or organisms belonging to the genus *Schizochytorium.*

Examples of the organisms belonging to the genus *Euglena* include *Euglena gracilis, Euglena longa, Euglena caudata, Euglena oxyuris, Euglena tripteris, Euglena proxima, Euglena viridis, Euglena sociabilis, Euglena ehrenbergii, Euglena deses, Euglena pisciformis, Euglena spirogyra, Euglena acus, Euglena geniculata, Euglena intermedia, Euglena mutabilis, Euglena sanguinea*, *Euglena stellata*, *Euglena terricola*, *Euglena klebsi*, *Euglena rubra*, and *Euglena cyclopicola.*

Examples of the *Euglena gracilis* include *Euglena gracilis* NIES-48 (strains stored in the Microbial Culture Collection at the National Institute for Environmental Studies, which will be described below).

Examples of the organisms belonging to the genus *Chlorella* include *Chlorella vulgaris*, *Chlorella pyrenoidosa*, and *Chlorella sorociniana.*

Examples of the *Chlorella sorociniana* include *Chlorella sorociniana* NIES-2169 (strains stored in the Microbial Culture Collection at the National Institute for Environmental Studies, which will be described below).

Examples of the organisms belonging to the genus *Auxenochlorella* include *Auxenochlorella protothecoides.*

Examples of the organisms belonging to the genus *Botryococcus* include *Botryococcus braunii.*

Examples of the organisms belonging to the genus *Nannochloris* include *Nannochloris bacillaris* and *Nannochloris normandinae.*

Examples of the organisms belonging to the genus *Nannochloropsis* include *Nannochloropsis oculata.*

Examples of the organisms belonging to the genus *Neochloris* include *Neochloris aquatica*, *Neochloris cohaerens*, *Neochloris conjuncta*, *Neochloris gelatinosa*, *Neochloris pseudostigmata*, *Neochloris pseudostigmatica*, *Neochloris pyrenoidosa*, *Neochloris terrestris*, *Neochloris texensis*, *Neochloris vigensis*, *Neochloris wimmeri*, and *Neochloris oleoabundans.*

Examples of the organisms belonging to the genus *Pseudochoricystis* include *Pseudochoricystis ellipsoidea.*

Examples of the organisms belonging to the genus *Scenedesmus* include *Scenedesmus ovaltermus*, *Scenedesmus disciformis*, *Scenedesmus acumunatus*, and *Scenedesmus dimorphus.*

Examples of the organisms belonging to the genus *Aurantiochytrium* include *Aurantiochytrium limacinum* and *Aurantiochytrium mangrovei.*

Examples of the organisms belonging to the genus *Schizochytorium* include *Schizochytrium aggregatum.*

The aforementioned microalgae are easily available, for example, from the National Institute of Technology and Evaluation at the NITE Patent Microorganisms Depositary (Kazusakamatari 2-5-8, Kisarazu, Chiba 292-0818 Japan), the Microbial Culture Collection at the National Institute for Environmental Studies (Onogawa 16-2, Tsukuba, Ibaraki 305-8506 Japan), or the Culture Collection of Algae at the University of Texas at Austin, USA (http://web.biosci.utexas.edu/utex/default.aspx).

The microalgae are preferably the organisms belonging to the genus *Chlorella* in that mass production of triglyceride that serves as a raw material of biodiesel is possible, valuables such as dietary fibers, vitamins, carotenoids, proteins, linoleic acid, and linolenic acid are contained in a large amount, and mass cultivation thereof is easy.

Further, the microalgae are preferably the organisms belonging to the genus *Euglena* in that mass production of wax esters that serve as a raw material of biodiesel is possible, valuables such as vitamins, carotenoids, proteins whose nutritional value is high, and paramylum are contained in a large amount, and mass cultivation thereof is easy.

The brewed beverage is produced by alcoholic fermentation of a raw material containing sugar by yeast, and is undistilled. That is, the brewed beverage is an undistilled liquid obtained by alcoholic fermentation of a raw material containing sugar by yeast and removing solid contents therefrom.

It should be noted that the sugar means at least one of monosaccharides and disaccharides.

The brewed beverage is not distilled and contains metabolites of alcoholic fermentation by yeast, and thus it contains various components produced by the yeast other than ethanol and water.

Specifically, the brewed beverage, for example, contains saccharides such as glucose, proteins, amino acids, vitamins, phosphorus, and potassium. These components of the brewed beverage can promote the growth of the microalgae during cultivation.

Examples of the brewed beverage include sake, wine, beer, brewed beverages using grains as a raw material, brewed beverages using legumes as a raw material, brewed beverages using potatoes as a raw material, and brewed beverages using sugar as a raw material.

The brewed beverage is preferably at least one selected from the group consisting of beer, sake, and wine.

The beer is produced by saccharification of starch contained at least in malt with enzyme contained in the malt so as to produce sugar and further alcoholic fermentation of the sugar by beer yeast. That is, products using still other raw materials are included in the beer in this description, as long as they are produced as described above at least using malt as a raw material. As the yeast, beer yeast is generally used.

As the malt, barley malt is generally used.

Examples of the beer include so-called beer, low-malt beer, other effervescent alcoholic beverages (the so-called third beer such as other brewed beverages or liquors), which are classified by the Japanese Liquor Tax Act. The beer is produced using a fermentation product of a raw material that can undergo alcoholic fermentation.

In the classification by the Japanese Liquor Tax Act, the so-called beer means fermentation products, using malt, hop, and water as raw materials, or fermentation products, using malt, hop, and water, and further wheat, rice, corn, kaoliang, potato, starch, or saccharides as raw materials, with an alcohol content of less than 20 degrees and a malt use rate of 66.7% (two out of three) or more.

In the classification by the Japanese Liquor Tax Act, the low-malt beer means effervescent products, using malt or wheat as part of raw materials, with an alcohol content of less than 20 degrees and a malt use rate of less than 66.7%. Further, the so-called third beer (new genre) means effervescent products other than the so-called beer and low-malt beer, as classified by the Japanese Liquor Tax Act, with an alcohol content of less than 10 degrees. The so-called third beer is classified as other effervescent alcoholic beverages by the Japanese Liquor Tax Act. Examples of the third beer include products using saccharides, hop, water, or the like as raw materials, or products obtained by adding spirits to the low-malt beer. These examples of the so-called beer, the low-malt beer, and the other effervescent alcoholic beverages can be used as a brewed beverage in the method for culturing microalgae.

The sake (Japanese sake) is produced by saccharification of starch contained in rice with rice malt so as to produce sugar and further alcoholic fermentation of the sugar by yeast. As the yeast, sake yeast is generally used.

As the rice malt, malted rice is generally used.

The wine is produced at least by alcoholic fermentation of grape juice by yeast. As the yeast, wine yeast is generally used.

The grape juice contains sugar such as glucose, and the sugar contained in the grape juice is transformed into ethanol due to the alcoholic fermentation by yeast.

Examples of the wine include red wine and white wine.

Examples of the brewed beverages using grains as a raw material include products obtained, using grains such as corn and kaoliang as a raw material, by saccharification of polysaccharides contained in the raw material so as to obtain sugar and alcoholic fermentation of the sugar by yeast.

Examples of the brewed beverages using legumes as a raw material include products obtained, using soybeans, adzuki beans, mung beans, common beans, peanuts, peas, or broad beans as a raw material, by saccharification of polysaccharides contained in the raw material so as to obtain sugar and alcoholic fermentation of the sugar by yeast.

Examples of the brewed beverages using potatoes as a raw material include products obtained, using potatos, sweet potatos, or the like, as a raw material, by saccharification of polysaccharides contained in the raw material so as to obtain sugar and alcoholic fermentation of the sugar by yeast.

Examples of the brewed beverage using sugar as a raw material include products obtained, using juice of sugar cane or sugar beet as a raw material, by alcoholic fermentation of sucrose contained in the raw material by yeast.

In the culture method, it is preferable that the liquid contain the brewed beverage so that ethanol contained in the brewed beverage is 0.5 vol% or more, and further it is preferable that the liquid contain the brewed beverage so that the ethanol exceeds 1.0 vol%.

When the liquid contains the brewed beverage so that ethanol contained in the brewed beverage is 0.5 vol% or more, there is an advantage that the growth of microalgae is further enhanced.

In particular, when sake is used as the brewed beverage, it is preferable that the liquid contain the sake so that ethanol contained in the sake exceeds 1.0 vol%. When the liquid contains the sake so that ethanol derived from the sake exceeds 1.0 vol%, there is an advantage that organic nutrients that can be used by the microalgae are allowed to exist more reliably within the liquid.

On the other hand, in the culture method, it is preferable that the liquid contain the brewed beverage so that ethanol contained in the brewed beverage is 3.0 vol% or less. When the liquid contains the brewed beverage so that ethanol contained in the brewed beverage is 3.0 vol% or less, there is an advantage that inhibition of the growth of microalgae by ethanol is more suppressed.

As described above, in the method for culturing microalgae of this embodiment, the microalgae are cultured in a liquid at least containing a brewed beverage, and therefore the microalgae can be grown efficiently due to organic nutrients such as ethanol, proteins, amino acids, saccharides such as glucose, and vitamins, or inorganic nutrients such as phosphorus and potassium, which are contained in the brewed beverage.

The sweet wort that can be used in the culture method of this embodiment is a liquid product generated by immersing malt in water so as to saccharify starch contained in the malt with enzyme contained in the malt. The beer can be produced by allowing sugar contained in the sweet wort to undergo alcoholic fermentation by beer yeast.

The sake lees are solid contents in mush (moromi) generated during the process for producing the sake. That is, the mush (moromi) from which the solid contents are removed serves as the sake.

The sake lees have components such as saccharides such as glucose, proteins, amino acids, phosphorus, potassium, iron, and magnesium.

The shochu lees are residues after the distillation process of mush (moromi) generated by alcoholic fermentation of at least one of grains, potatos, and brown sugar by yeast.

Examples of the grains as a raw material of the shochu lees include rice, barley, wheat, and buckwheat.

The shochu lees have components such as proteins, amino acids, citric acid, malic acid, succinic acid, phosphorus, potassium, and vitamins.

The yeast is a heterotrophic organism that is a kind of eukaryote and is unicellular and aplanetic, having cell walls and no photosynthetic capability.

The yeast can generally perform alcoholic fermentation.

Examples of the yeast include organisms belonging to the genus *Saccharomyces*, specifically, *Saccaromyces cerebisiae.*

Further, examples of the yeast include so-called sake yeast, so-called wine yeast, and so-called beer yeast, depending on its application.

As the yeast, organisms whose cell walls are broken by contact with hot water, or organisms whose cell walls are broken by enzyme treatment also can be used.

The yeast autolysate (hereinafter, referred to also as yeast extract) is generated by autolysis of the yeast. That is, the yeast autolysate is generated by decomposition of components constituting the yeast using enzyme contained in the yeast.

The autolysis of the yeast, for example, can be caused by placing the yeast in the absence of organic nutrients such as saccharides.

The yeast autolysate has components such as proteins, amino acids, vitamins, phosphorus, and potassium.

When the yeast autolysate is mixed with the liquid, the concentration of the yeast autolysate in the liquid is preferably 0.2 vol% or less, more preferably 0.1 vol% or less, since the yeast autolysate can sufficiently promote the growth of the microalgae without having a higher concentration.

The blackstrap molasses are residues after the process for extracting sucrose from the juice of sugar cane or sugar beet.

The blackstrap molasses contains sucrose, glucose, fructose, potassium, and the like.

Further, in the method for culturing microalgae of this embodiment, it is possible to allow photoautotrophic microalgae that are capable of photosynthesis to perform photosynthesis by irradiation of the microalgae with light. That is, in the culture method, it is possible to cause the microalgae to perform photosynthesis, while growing the microalgae using nutrients contained in the brewed beverage.

When the microalgae are irradiated with light in the culture method, the photoautotrophic microalgae that are capable of photosynthesis can grow using organic nutrients (for example, proteins or amino acids contained in the brewed beverage) in the liquid as a nutrient source, while synthesizing hydrocarbons or saccharides by incorporating carbon dioxide into their cells by photosynthesis.

The light with which the microalgae are irradiated in the culture method is not specifically limited, as long as it causes the microalgae to perform photosynthesis. For example, natural sunlight or artificial light such as illumination light can be employed as the light.

In this culture method, while the microalgae are grown in the liquid containing the brewed beverage, a time period during which the microalgae are irradiated with light and a time period during which the microalgae are not irradiated with light can be alternately provided.

That is, in the culture method, a time period during which the microalgae are grown while the microalgae are irradiated with light so as to perform photosynthesis and a time period during which the microalgae are grown while photosynthesis is suppressed under dark conditions can be alternately repeated.

The time period of irradiation with light in the culture method is not specifically limited, and is generally 8 hours to 15 hours, which is equivalent to daytime during which sunlight shines. Further, the time period under dark conditions during which the microalgae are substantially prevented from performing photosynthesis is generally 9 hours to 16 hours, which is equivalent to night-time during which sunlight does not shine. These periods can be changed depending on the situation or purpose.

In the aforementioned culture method, the microalgae may be irradiated with light for 24 hours per day. That is, the microalgae may be allowed to perform photosynthesis continuously during the cultivation.

Brightness of the light for irradiation in the culture method is not specifically limited. However, particularly in the case of culturing organisms belonging to the genus *Euglena* as microalgae, the light preferably has an intensity of 50 µmol/m²/s to 200 µmol/m²/s.

When the light intensity is 50 µmol/m²/s or more, there is an advantage that the photosynthesis can be further promoted. Further, when the light intensity is 200 µmol/m²/s or less, there is an advantage that inhibition of the growth due to the light can be more reliably suppressed.

On the other hand, in the culture method, the microalgae may be grown while the microalgae are not irradiated with light so that the photosynthesis of photoautotrophic microalgae that are capable of photosynthesis is suppressed. That is, the microalgae may be grown under dark conditions.

In the culture method, in the case of employing the photoautotrophic microalgae that are capable of photosynthesis as the microalgae, the microalgae are preferably irradiated with light since the growth of the microalgae can be further promoted.

Specifically, in the culture method, in the case of employing organisms belonging to the genus *Euglena* as the microalgae, the organisms belonging to the genus *Euglena* synthesize organic compounds (polysaccharides, lipids, or the like) from carbon dioxide by photosynthesis so as to store the organic compounds within their cells while growing under the conditions of being irradiated with light. On the other hand, the organisms belonging to the genus *Euglena* synthesize organic compounds (polysaccharides, lipids, or the like) from organic nutrients contained in the liquid so as to store the organic compounds within their cells while growing under the dark conditions without being irradiated with light. The organisms belonging to the genus *Euglena* that have stored the organic compounds, for example, are collected, and can be directly used as valuables.

The culture temperature in the culture method is not specifically limited, as long as it allows the microalgae to grow. A culture temperature (temperature of the liquid) of 20°C to 35°C, for example, is employed.

The pH of the liquid in the culture method is not specifically limited, as long as it allows the microalgae to grow. A pH of 3.0 to 5.5, for example, is employed in the case where the organisms belonging to the genus *Euglena* are cultured.

For adjusting the pH of the liquid, an inorganic acid such as hydrochloric acid may be added to the liquid, or an organic acid such as acetic acid may be added to the liquid. Further, an inorganic acid and an organic acid may be used in combination. The addition of an organic acid to the liquid allows the microalgae to grow using the organic acid as an organic nutrient.

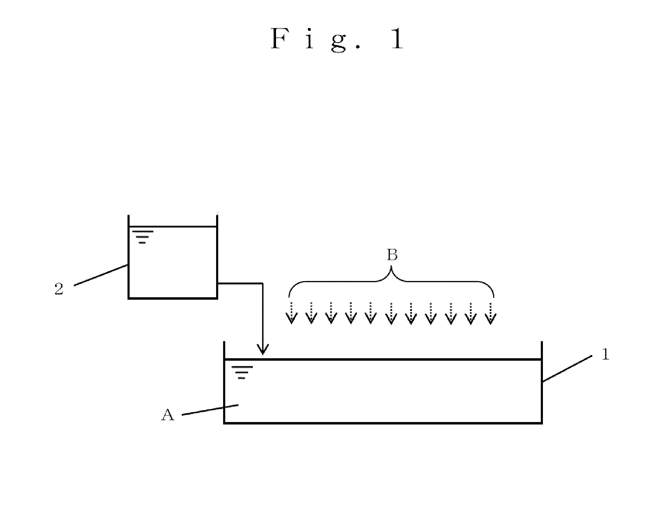

In the culture method, a culture tank 1 for accommodating the microalgae and a liquid A for culturing the microalgae, for example, can be used as shown in Fig. 1. Further, a preparation tank 2 for preparing a mixed solution in which the brewed beverage is mixed at least with water can be used.

The culture tank 1 includes a stirring device, and is configured so that the microalgae and the liquid A are stirred within the tank.

The culture tank 1 may be formed so as to have a comparatively small depth so that light B emitted from above can reach the bottom transmitting through the liquid A, in order to promote photosynthesis of the microalgae accommodated therein.

On the other hand, in the case where the microalgae are cultured only by heterotrophic cultivation while photosynthesis of the microalgae is suppressed, the culture tank 1 may be formed so as to have a comparatively large depth so that oxygen for oxygen breathing of the microalgae can be easily dissolved in the liquid A.

The culture tank 1 may be configured to supply a gas containing at least one of oxygen and carbon dioxide to the liquid A accommodated therein.

The liquid A generally contains water. Further, most part of the liquid A accommodated in the culture tank 1 is generally water.

The preparation tank 2 is configured to supply a mixture of water, a brewed beverage, and at least one of sweet wort, sake lees, shochu lees, yeast, yeast autolysate, and blackstrap molasses to the culture tank 1 so that the liquid A accommodated in the culture tank 1 contains suitable amounts of the brewed beverage and the at least one of sweet wort, sake lees, shochu lees, yeast, yeast autolysate, and blackstrap molasses.

That is, in the culture method, the brewed beverage and the at least one of sweet wort, sake lees, shochu lees, yeast, yeast autolysate, and blackstrap molasses are supplied from the preparation tank 2 to the culture tank 1, as shown in Fig. 1, so that the microalgae are grown in the liquid A in the culture tank 1.

As shown in Fig. 1, the culture tank 1 is configured to allow the microalgae in the liquid A accommodated therein to be irradiated with the light B.

The light B for irradiation is not specifically limited, as long as it causes photosynthesis of the microalgae. For example, natural sunlight or artificial light such as illumination light can be employed as the light B, as mentioned above.

In the culture method of this embodiment, a gas containing oxygen can be supplied to the liquid A, in order to maintain oxygen breathing of the microalgae. Further, in the culture method, a gas containing carbon dioxide can be supplied to the liquid A, in order to promote the photosynthesis of photoautotrophic microalgae that are capable of photosynthesis.

Such a gas can be supplied by aerating the liquid A accommodated in the culture tank 1, or stirring the liquid A.

Specifically, in the culture method, the liquid A can be aerated, for example, with air, in order to supply oxygen for breathing to the microalgae. Further, in the culture method, the liquid A can be aerated, for example, with exhaust gas containing a comparatively large amount of carbon dioxide, in order to promote the photosynthesis of the microalgae.

In the culture method, the gas may be supplied to the liquid A in the culture tank 1 on a constant basis, or the gas may be supplied to the liquid A in the culture tank 1 only when the microalgae are cultured under the dark conditions.

As a method for supplying the gas to the liquid A in the culture tank 1, using an aeration tube, stirring the liquid A with stirring blades so that oxygen or carbon dioxide is dissolved in the liquid A, or supplying pressurized water in which oxygen or carbon dioxide is pressurized so as to be dissolved therein to the liquid A can be employed.

In the culture method, light heterotrophic cultivation is preferably performed while oxygen and carbon dioxide are supplied to the liquid A by aeration or the like. That is, in the culture method, it is preferable that the microalgae be caused to perform photosynthesis by irradiation with light, while heterotrophic cultivation is performed in the liquid A at least containing a brewed beverage, and a gas containing both of oxygen and carbon dioxide is supplied to the liquid A by aeration or the like, in order to promote oxygen breathing and photosynthesis of the microalgae capable of photosynthesis.

When the culture method is implemented as above, there are advantages that the growth of the microalgae capable of photosynthesis is further promoted, and production of organic compounds by the microalgae is further promoted.

Specifically, in the culture method, the microalgae capable of photosynthesis are caused to perform photosynthesis, for example, in the liquid A containing a brewed beverage while the liquid A is aerated with a gas containing oxygen and carbon dioxide, so as to be cultured by heterotrophic cultivation (light heterotrophic cultivation) using organic nutrients contained in the liquid A. In such cultivation, the growth speed of the microalgae is higher than in simple heterotrophic cultivation, which will be described below, even with a light-irradiation time, for example, of about 8 hours. This is probably because, in heterotrophic cultivation with aeration, while the microalgae incorporate components of the brewed beverage that are easy to incorporate, the microalgae perform photosynthesis, which allows the microalgae to incorporate components of the brewed beverage that are difficult to incorporate into their cells so as to metabolize them by a metabolic system different from oxygen breathing. In the culture method, a liquid in which carbon dioxide is dissolved may be used.

On the other hand, in the culture method, the microalgae can be grown while the microalgae are placed in dark conditions without being irradiated with the light B, and a gas containing oxygen is supplied to the liquid A, thereby allowing the microalgae to use organic nutrients contained in the liquid A (for example, ethanol or proteins contained in the brewed beverage) as a nutrient source.

Specifically, in the culture method, the microalgae can be cultured (heterotrophic cultivation) in the liquid A containing organic nutrients contained in the brewed beverage, while the liquid A is aerated with a gas containing oxygen.

In such cultivation, if the microalgae use only glucose that is an organic nutrient as a nutrient, the growth of microalgae can become comparatively rapid because glucose is a nutrient that is easy for the microalgae to incorporate into their cells and thus is easy to use. On the other hand, when the microalgae use a brewed beverage as a material containing nutrients, as in the aforementioned culture method, physiological phenomena of the microalgae can be promoted by various nutrients, though the brewed beverage contains other nutrients that are more difficult to use as nutrients than glucose. The decrement in growth efficiency due to such nutrients that are difficult to use can be compensated for, for example, by photosynthesis of microalgae capable of photosynthesis.

In contrast, in the culture method, the liquid A may be placed in anaerobic conditions by stopping the supply of a gas to the liquid A.

For example, in the culture method, the organisms belonging to the genus *Euglena* that have stored organic compounds such as polysaccharides and lipids within their cells are placed in anaerobic conditions, thereby allowing the organisms belonging to the genus *Euglena* to store wax esters or the like within their cells. The stored wax esters can be used as a raw material for fuels or the like by being taken out of the cells of the organisms belonging to the genus *Euglena.*

Generally, in order to allow the organisms belonging to the genus *Euglena* to store wax esters, anaerobic conditions are provided by stopping aeration or supplying an inert gas free from oxygen to the liquid A, and dark conditions without irradiation with the light B are provided at the same time.

In the culture method of this embodiment, the liquid A at least contains water and a brewed beverage, and can further contain at least one of sweet wort, sake lees, shochu lees, yeast, yeast autolysate, and blackstrap molasses. The brewed beverage and the other components contain at least one of inorganic nutrients and organic nutrients as mentioned above.

In the culture method of this embodiment, nutrients such as inorganic nutrients or organic nutrients can be further added to the liquid A.

Examples of the inorganic nutrients (contained in the brewed beverage or further added) to be used include nitrogen-containing inorganic compounds that contain N and phosphorus-containing inorganic compounds that contain P. Further, examples of the inorganic nutrients to be used include potassium ions, iron ions, manganese ions, cobalt ions, zinc ions, copper ions, molybdenum ions, and nickel ions.

Examples of the organic nutrients (contained in the brewed beverage or further added) to be used include saccharides such as glucose, amino acids, ethanol, and vitamins.

In the culture method, the brewed beverage and sweet wort, sake lees, shochu lees, yeast, yeast autolysate, blackstrap molasses, or the like, which are contained in the liquid A, contain the aforementioned inorganic nutrients or organic nutrients, and inorganic nutrients or organic nutrients are further mixed in the liquid A, as needed, thereby allowing these nutrients to be used advantageously for cultivation of the microalgae. Thus, the growth of the microalgae can be promoted.

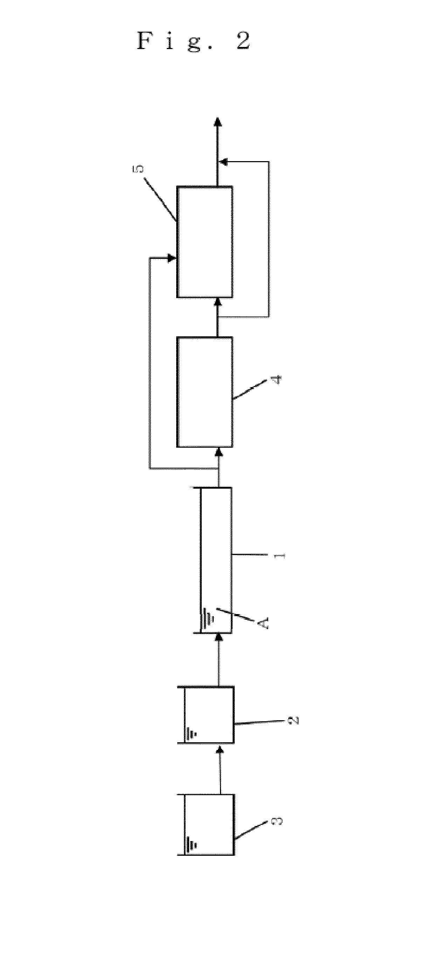

In the culture method, the liquid A can be allowed to further contain the inorganic nutrients or organic nutrients, for example, by dissolving inorganic nutrients or organic nutrients to be further added in water in the preparation tank 2 and thereafter delivering the aqueous solution in which the inorganic nutrients or organic nutrients are dissolved to the liquid A in the culture tank 1, as shown in Fig. 1 and Fig. 2.

Further, in the culture method, a sterilizing tank 3 for sterilization of materials to be supplied to the preparation tank 2, for example, can be used, as shown in Fig. 2.

The sterilizing tank 3 is configured so as to perform sterilization of water, the sweet wort, the yeast, or the like, within the tank. The sterilization can be performed, for example, by heating using the heat of water vapor, or the like, or filtration using a filtration membrane so as to remove bacteria.

In the culture method, for example, after materials other than the microalgae that are necessary for cultivation are sterilized by being added to the sterilizing tank 3, the sterilized materials may be supplied to the culture tank 1 via the preparation tank 2.

Alternatively, in the culture method, while at least one or all of nutrients to be further added to the liquid A, yeast, sweet wort, and water are added to the sterilizing tank 3, a brewed beverage such as beer and sake may be supplied to the preparation tank 2 without being supplied to the sterilizing tank 3. Then, after the materials supplied from the sterilizing tank 3 are mixed with the brewed beverage in the preparation tank 2, and the mixture may be supplied to the culture tank 1.

When the brewed beverage is not supplied to the sterilizing tank 3 and thus is not sterilized, there is an advantage that vitamins contained in the brewed beverage are not inactivated in the cultivation of the microalgae. Further, when the brewed beverage is not supplied to the sterilizing tank 3 and thus is not sterilized, there is an advantage that the volume to be accommodated in the sterilizing tank 3 is reduced by the amount that the brewed beverage is not supplied to the sterilizing tank 3, so that the energy necessary for sterilization in the sterilizing tank 3 can be reduced.

Further, in the culture method, an autolysis tank for autolysis of the yeast as a pretreatment can be used.

The autolysis tank is configured to accommodate yeast therein and expose the yeast to an environment for autolysis. Further, the autolysis tank is configured to supply yeast autolysate generated by the autolysis to the preparation tank 2 or the sterilizing tank 3.

The autolysis tank is configured to allow the yeast to autolyze, for example, by placing the yeast under conditions without nutrient sources such as saccharides at room temperature, or placing the yeast under conditions without nutrient sources after heating at 40 to 60°C. The autolysis tank is preferably configured to allow the yeast to autolyze under anaerobic conditions.

In the aforementioned culture method, an aqueous solution containing the yeast autolysate can be delivered to the liquid A in the culture tank 1 via the preparation tank 2 or the sterilizing tank 3 by using the autolysis tank.

In the method for culturing microalgae of this embodiment, it is preferable that the microalgae be cultured in facilities for producing beer as a brewed beverage and in a liquid containing beer generated in the facilities.

In the method for culturing microalgae of this embodiment, it is more preferable that the microalgae be cultured in facilities for producing beer as a brewed beverage and in a liquid containing beer generated in the facilities and at least one of sweet wort, yeast, and yeast autolysate that are generated in the facilities.

The facilities for producing beer include equipment for producing beer and a site for the equipment. The facilities for producing beer, for example, include a beer factory and a place adjacent to the beer factory. The facilities for producing beer include not only buildings but also the outdoors as long as they are places for producing beer.

The same applies also to facilities for producing sake, facilities for producing shochu, facilities for producing wine, and facilities for producing sucrose, which will be described below.

The method for culturing microalgae of this embodiment can be implemented, for example, in a beer factory or a place adjacent to the beer factory, as described below.

That is, in the method for culturing microalgae of this embodiment, the culture tank 1 is installed in a beer factory or a place adjacent to the beer factory, and beer discharged from the beer factory is used as the brewed beverage, thereby allowing the microalgae to be cultured in the liquid A within the culture tank 1.

In the culture method, the microalgae are cultured in a beer factory or a place adjacent to the beer factory, and the microalgae can be cultured, for example, in the liquid A containing wastewater containing beer discharged from the beer factory by using the wastewater.

Further, in the culture method, waste beer determined as non-standard products in the beer factory or expired beer returned to the beer factory, for example, is used for promoting the growth of the microalgae.

Further, in the culture method, sweet wort generated in the process for producing beer, yeast (beer yeast) for producing or after producing beer, or yeast autolysate generated by autolysis of the yeast, for example, is used for promoting the growth of the microalgae.

Further, in the culture method, exhaust gas containing a comparatively large amount of carbon dioxide discharged from a boiler or the like for heating in the beer factory, for example, can be employed as a supply source of carbon dioxide in the aforementioned cultivation together with photosynthesis of the microalgae (cultivation under bright conditions).

As described above, the microalgae can be cultured only by using materials discharged from the factory (such as wastewater, beer yeast, and waste heat) by implementing the method for culturing microalgae in a beer factory or a place adjacent to the beer factory. Further, energy is extracted from the microalgae obtained by the cultivation, so that the energy can be used for operating the beer factory.

As a method for extracting energy from the microalgae, a method in which energy is directly extracted by drying the cultured microalgae and using the dried microalgae themselves as fuels, or a method in which energy is indirectly extracted by extracting organic compounds such as oils from the cultured microalgae and using the organic compounds as fuels or the like can be employed.

In the culture method that uses beer as mentioned above, the microalgae are cultured in the liquid A in which the beer is diluted preferably to a concentration of 2 to 10 times, more preferably to 2 to 5 times. When the microalgae are cultured in the liquid A in which the beer (waste beer or returned beer) is diluted to a concentration of 2 to 10 times, it is possible to suppress inhibition of the growth of the microalgae due to alcohol, while preventing a reduction in the growth efficiency of the microalgae due to lack of nutrients.

As dilution water with which the beer is diluted, the aforementioned wastewater containing beer discharged from the beer factory can be used. Further, as dilution water with which the beer is diluted, water such as tap water used in the beer factory, industrial water, washing water for beer bottles or the like, and treated water in wastewater treatment equipment can be used.

As the dilution water, water free from sterilization components such as sodium hypochlorite, water in which sterilization components are inactivated, or water in which sterilization components are diluted to an effective concentration or less is preferably used.

Further, in the culture method that uses beer as mentioned above, carbon dioxide dissolved in the beer can be used. That is, use of microalgae capable of photosynthesis as the microalgae and use of beer in which carbon dioxide is dissolved as the brewed beverage can promote the growth of the microalgae that perform photosynthesis. When the liquid A containing carbon dioxide is aerated, the liquid A might become excessively effervescent. Therefore, in the case of growing the microalgae while aerating the liquid A containing carbon dioxide dissolved in beer, the beer may be subjected to degassing treatment as a pretreatment, in order to suppress the excessive effervescence of the liquid A due to carbon dioxide. The degassing treatment can be performed by stirring, heating, filtration, or the like.

Further, in the culture method that uses beer as mentioned above, the microalgae are cultured only by using materials discharged from the beer factory as materials containing nutrients to be contained in the liquid A. Therefore, the growth of the microalgae is less likely to be inhibited, since it is difficult for substances that inhibit the growth of the microalgae to enter therein from the outside. Further, contaminants are less likely to incorporate into the grown microalgae from the outside. Therefore, when using the microalgae as valuables (for example, health foods or chemical products), it is possible to suppress contamination of the valuables due to contaminants.

In the method for culturing microalgae of this embodiment, it is preferable that the microalgae be cultured in facilities for producing sake as a brewed beverage and in a liquid containing sake generated in the facilities.

In the method for culturing microalgae of this embodiment, it is more preferable that the microalgae be cultured in facilities for producing sake as a brewed beverage and in a liquid containing sake generated in the facilities and at least one of sake lees, yeast, and yeast autolysate that are generated in the facilities.

The method for culturing microalgae of this embodiment can be implemented, for example, in a sake factory or a place adjacent to the sake factory, in the same manner as the culture method which is implemented in the facilities for producing beer as mentioned above. In the culture method, sake, sake lees generated in the process for producing the sake, yeast, and yeast autolysate generated by autolysis of the yeast, for example, are used as materials containing nutrients for promoting the growth of the microalgae.

In the culture method that uses sake or the like as mentioned above, the microalgae are cultured in the liquid A in which the sake is diluted preferably to a concentration of 5 to 30 times, more preferably to 5 to 15 times.

In the sake factory, components of wastewater largely vary due to the season, and thus a large load is applied to the wastewater treatment equipment in the factory depending on the season, for the comparatively small scale of the factory. Accordingly, by implementing the culture method in the sake factory or the place adjacent to the sake factory, using sake that is about to be disposed of and sent to the wastewater treatment equipment, variation of the load applied to the wastewater treatment equipment can be suppressed, and further the microalgae can be grown. Furthermore, the grown microalgae can be used as valuables.

In the method for culturing microalgae of this embodiment, the microalgae can be cultured, for example, in facilities for producing shochu. In the culture method, the microalgae can be cultured in a liquid, for example, containing a brewed beverage and at least one of shochu lees, yeast, and yeast autolysate that are generated in the facilities.

The culture method can be implemented, for example, in a shochu factory or a place adjacent to the shochu factory in the same manner as the culture method which is implemented in the facilities for producing beer as mentioned above. In the culture method, shochu lees generated in the process for producing the shochu, yeast, and yeast autolysate generated by autolysis of the yeast, for example, are used as materials for promoting the growth of the microalgae.

In the method for culturing microalgae of this embodiment, it is preferable that the microalgae be cultured in facilities for producing wine as a brewed beverage and in a liquid containing wine generated in the facilities.

In the method for culturing microalgae of this embodiment, it is more preferable that the microalgae be cultured in facilities for producing wine as a brewed beverage and in a liquid containing wine generated in the facilities and at least one of yeast generated in the facilities and yeast autolysate.

The method for culturing microalgae of this embodiment can be implemented in the facilities for producing wine. The culture method can be implemented, for example, in a wine factory or a place adjacent to the wine factory in the same manner as the culture method which is implemented in the facilities for producing beer as mentioned above.

In the culture method, wine, yeast generated in the process for producing the wine, and yeast autolysate generated by autolysis of the yeast, for example, are used as materials for promoting the growth of the microalgae.

The method for culturing microalgae of this embodiment can be implemented in facilities for producing sucrose. The culture method can be implemented, for example, in a sugar factory or a place adjacent to the sugar factory in the same manner as the culture method which is implemented in the facilities for producing beer as mentioned above. In the culture method, the microalgae can be cultured in a liquid, for example, containing a brewed beverage and blackstrap molasses generated in the process for producing sucrose (sugar refining process).

In the method for culturing microalgae of this embodiment, the microalgae are cultured as described above, thereby allowing the microalgae to grow, so that at least one of organic compounds such as hydrocarbons, oils and fats, and polysaccharides can be stored within the cells of the microalgae.

Then, the microalgae with these organic compounds stored within the cells can be used as biomass that can be used for various applications such as foods, drugs, feeds, chemical products, and fuels.

Specifically, in the case where the organisms belonging to the genus *Euglena* are employed as the microalgae, cultivation under aerobic conditions or cultivation together with photosynthesis under light irradiation conditions allows paramylum to be generated within the cells so as to be extracted and refined for use.

In the mixture of the microalgae grown by cultivation in the culture tank 1 and the liquid A, the concentration of the microalgae is increased, for example, by using a concentrating device 4 that applies concentration treatment to the microalgae and/or a dehydrating device 5 that applies dehydration treatment to the microalgae, as shown in Fig. 2. As shown in Fig. 2, the mixture of the microalgae and the liquid A may pass through only the concentrating device 4, may pass through only the dehydrating device 5, or may pass through both the concentrating device 4 and the dehydrating device 5. The microalgae that have passed through the concentrating device 4 and/or the dehydrating device 5 can be used as biomass, as described above.

Examples of the concentrating device 4 include devices configured to concentrate the microalgae, for example, by elevated concentration, gravity concentration, membrane concentration, and belt concentration.

Further, examples of the dehydrating device 5 include a vacuum dewatering device, a pressure dewatering device (filter press), a belt press, a screw press, a centrifugal dewatering device (screw decanter), and a multi-disk dewatering device.

Subsequently, an embodiment of a method for using microalgae according to the present invention is described.

The method for using microalgae of this embodiment uses the microalgae obtained by the aforementioned method for culturing microalgae at least as fuels in the facilities in which the culture method has been implemented.

The organic compounds that the microalgae have stored within their cells in the culture method can be efficiently used by implementing the method for using microalgae of this embodiment.

In the method for using microalgae, specific examples of using the microalgae as fuels in the facilities are as described above.

Further, the cultured microalgae can be used in the facilities for applications such as chemical products other than fuels.

The method for culturing microalgae and the method for using microalgae of the aforementioned embodiments are as exemplified above. However, the present invention is not limited to the method for culturing microalgae and the method for using microalgae exemplified above.

Further, various embodiments generally used in methods for culturing microalgae and methods for using microalgae can be employed, as long as the effects of the present invention are not impaired.

For example, in the method for culturing microalgae of the present invention, a batch culture method in which all the cultured microalgae are extracted from the culture tank 1 after every cultivation may be employed using the culture tank 1. Further, a semi-batch culture method in which the cultured microalgae are partially extracted from the culture tank 1 may be employed. Furthermore, a continuous culture method in which, while the microalgae are partially continuously extracted from the culture tank 1 with the growth speed of the microalgae, nutrients are continuously added to the liquid A in the culture tank may be employed.

Further, in the method for culturing microalgae of the present invention, in the case where the capacity of the culture tank 1 is very large, microalgae cultured in advance in another culture tank (pretreatment culture tank) may be supplied to the culture tank 1 so that the microalgae are further cultured in the culture tank, in order to increase microalgae in the culture tank 1 to a specific concentration or more. Such cultivation in which microalgae are put into the culture tank 1 after the microalgae are increased in advance to a specific concentration or more can reduce the culture time in the culture tank 1. In particular, in the case of cultivation using waste beer as mentioned above, the amount of waste beer is comparatively small, and therefore it is effective to culture microalgae in another culture tank (pretreatment culture tank) in advance, as mentioned above, for improving the culture efficiency in the culture tank 1.

In the cultivation of microalgae in another culture tank (pretreatment culture tank), microalgae can be grown using common cultivation nutrients under conditions without irradiation with the light B, while aeration with a gas containing oxygen is performed. This allows the microalgae to grow efficiently, even if the size of the other culture tank (pretreatment culture tank) is comparatively small, so that microalgae increased to a specific concentration or more can be cultured in the culture tank 1 in a subsequent step.

### EXAMPLES

Next, the present invention is described further in detail by way of examples. However, the present invention is not limited to these examples.

The following materials were prepared for culturing microalgae, which were cultured under the following conditions.
Microalgae: Organisms belonging to the genus *Euglena (Euglena gracilis* NIES-48), obtained from the Microbial Culture Collection at the National Institute for Environmental Studies
Culture container: 300-mL triangle flask
Shake culture conditions: 120 rpm (air was supplied to the liquid by shaking) Culture temperature: 28°C
Liquid pH: 4 (adjusted by hydrochloric acid)
Liquid composition:
A mixture (hereinafter, referred to also as [AF-6 composition]) of components shown in Table 1, which were mixed so as to have the same composition as "AF-6" (culture medium name), was used as the ingredients of the liquid.

The composition of "AF-6" is disclosed by the Microbial Culture Collection at National Institute for Environmental Studies, in which components shown in Table 1 are mixed in 99.5 mL of water.

Further, the content in the liquid other than nutrients is water. Specifically, a liquid containing water was prepared so that components of the liquid that are derived from the aforementioned AF-6 composition have a concentration shown in Table 1 below in 100 mL of the liquid. That is, the liquid containing water was adjusted so that ingredients derived from the AF-6 composition in 100 mL of the liquid in the cultivation respectively exhibit the values shown in Table 1. Specifically, the concentration of each liquid in the cultivation was adjusted so that, for example, in the case of NaNO₃, 14 mg of NaNO₃ was contained in 100 mL of the liquid.

**Table 1**

| | |
|---|---|
| NaNO₃ | 14 mg |
| NH₄NO₃ | 2.2 mg |
| MgSO₄·7H₂O | 3 mg |
| KH₂PO₄ | 1 mg |
| K₂HPO₄ | 0.5 mg |
| CaCl₂·2H₂O | 1 mg |
| Fe-citrate | 0.2 mg |
| Citric acid | 0.2 mg |
| Biotin | 0.2 µg |
| Thiamine HCl | 1 µg |
| Vitamin B₆ | 0.1 µg |
| Vitamin B₁₂ | 0.1 µg |
| Trace metal solution | 0.5 mL |

As a trace metal solution in Table 1, a trace metal solution having the composition shown in Table 2 below was used. The trace metal solution was mixed in the liquid in the amount shown in Table 1 in every cultivation.

Further, the composition shown in Table 2 is the same as the composition of "P IV metals" (culture medium name) disclosed by the Microbial Culture Collection at the National Institute for Environmental Studies. In the cultivation, a mixture (hereinafter, referred to also as P IV metal composition) of components, which were mixed so as to have the same composition as "P IV metals", was used.

As described above, the liquid was prepared in each test example described below so that the concentration of components of the liquid derived from the AF-6 composition and the concentration of components of the liquid derived from the P IV metal composition are respectively equal to the values shown in Table 1.

**Table 2**

| | |
|---|---|
| Na₂EDTA·2H₂O | 100 mg |
| FeCl₃·6H₂O | 19.6mg |
| MnCl₂·4H₂O | 3.6mg |
| ZnSO₄·H₂O | 2.2mg |
| CoCl₂·6H₂O | 0.4mg |
| Na₂MoO₄·2H₂O | 0.25mg |
| Distilled water | 100 mL |

Hereinafter, conditions for culturing the microalgae are further shown. Light irradiation conditions:
After light irradiation for 12 hours, left to stand in a dark place for 12 hours (hereinafter, referred to also as light conditions 1)
Left to stand in a dark place for 24 hours (hereinafter, referred to also as light conditions 2)
The light intensity of light irradiation was set to 100 µmol/m²/s.
   Initial weight of microalgae: 0.78 g/L (dry weight)
   Brewed beverage: Brewed beverages used are shown below.

### "Beer"

The following four types each have a malt use rate of 66.7% (two out of three) or more (categorized as beer under the Liquor Tax Act)
- Product name: Black Label, manufactured by Sapporo Breweries Limited, ethanol 5 vol%
- Product name: Super Dry, manufactured by Asahi Breweries, Ltd.
- Product name: Ichiban Shibori, manufactured by Kirin Brewery Company, Limited
- Product name: Malt's, manufactured by Suntory Holdings Limited

The following four types each have a malt use rate of less than 66.7%, in which spirits are added to low-malt beer under the Liquor Tax Act
(categorized as liquors under the Liquor Tax Act)
- Product name: Mugi-to-Hop, manufactured by Sapporo Breweries Limited, ethanol 5 vol%
- Product name: Clear Asahi, manufactured by Asahi Breweries, Ltd.
- Product name: Mugi-no-Gochisou, manufactured by Kirin Brewery Company, Limited
- Product name: Kinmugi, manufactured by Suntory Holdings Limited "Sake"
- Product name: Banshaku, manufactured by NIHONSAKARI Co., Ltd., ethanol 13 vol%
- Product name: Jousen Kinkan, manufactured by Ozeki Corporation
- Product name: Jousen Gekkeikan, manufactured by Gekkeikan Sake Company, Ltd.
- Product name: Jousen Hakutsuru, manufactured by HAKUTSURU SAKE BREWING CO.,LTD.
   "Yeast autolysate (yeast extract)"
- Product name: Dried Yeast Extract D-3, manufactured by NIHON PHARMACEUTICAL CO., LTD.

Microalgae were able to be cultured using any beer mentioned above as a brewed beverage. Further, microalgae were able to be cultured using any sake mentioned above as a brewed beverage.

The results when using "Black Label" (hereinafter, referred to as Beer 1) manufactured by Sapporo Breweries Limited (Test example 1), the results when using "Mugi-to-Hop" (hereinafter, referred to as Beer 2) manufactured by Sapporo Breweries Limited (Test example 2), and the results when using "Banshaku" manufactured by NIHONSAKARI Co., Ltd. (Test example 3) are shown below.

Specifically, in each test example, microalgae were cultured for 3, 5, or 7 days in a liquid containing each brewed beverage (in which beer was diluted to twice or 5 times with water, or sake was diluted to 5 times or 15 times with water) under light conditions 1 or light conditions 2 in the presence or absence of yeast autolysate. The dry weight of the microalgae after the cultivation was measured.

The lists of the results using Beer 1, Beer 2, and sake as a brewed beverage are respectively shown in Table 3 to Table 5. The numerical values in Table 3 to Table 5 each show the amount of microalgae in the liquid after the cultivation (dry weight: g/L).

**Table 3**

| (Test example 1) | | | | | |
|---|---|---|---|---|---|
| Unit: Dry weight g/L | | | | | |
| Beer 1 | | Concentration of yeast autolysate | Day 3 | Day 5 | Day 7 |
| Diluted to twice | Light conditions 1 | 0 vol% | 3.15 | 5.40 | 7.00 |
| | | 0.05 vol% | 3.45 | 7.40 | 10.60 |
| | | 0.1 vol% | 3.80 | 8.50 | 12.10 |
| | | 0.2 vol% | 3.10 | 7.00 | 10.70 |
| | Light conditions 2 | 0 vol% | 1.00 | 3.20 | 4.00 |
| | | 0.05 vol% | 1.30 | 2.80 | 4.80 |
| | | 0.1 vol% | 1.35 | 2.35 | 3.80 |
| | | 0.2 vol% | 1.45 | 2.45 | 3.50 |
| Diluted to 5 times | Light conditions 1 | 0 vol% | 3.35 | 5.60 | 5.40 |
| | | 0.05 vol% | 3.90 | 6.40 | 5.60 |
| | | 0.1 vol% | 4.65 | 6.80 | 6.20 |
| | | 0.2 vol% | 4.30 | 7.00 | 6.30 |
| | Light conditions 2 | 0 vol% | 2.75 | 4.20 | 4.90 |
| | | 0.05 vol% | 3.10 | 5.70 | 5.10 |
| | | 0.1 vol% | 3.35 | 5.10 | 4.70 |
| | | 0.2 vol% | 3.35 | 6.90 | 6.30 |

**Table 4**

| (Test example 2) | | | | | |
|---|---|---|---|---|---|
| Unit: Dry weight g/L | | | | | |
| Beer 2 | | Concentration of yeast autolysate | Day 3 | Day 5 | Day 7 |
| Diluted to twice | Light conditions 1 | 0 vol% | 2.85 | 4.60 | 5.60 |
| | | 0.05 vol% | 3.40 | 7.10 | 10.20 |
| | | 0.1 vol% | 3.40 | 7.00 | 10.90 |
| | | 0.2 vol% | 3.00 | 6.80 | 10.40 |
| | Light conditions 2 | 0 vol% | 1.30 | 3.00 | 3.90 |
| | | 0.05 vol% | 1.45 | 3.40 | 5.40 |
| | | 0.1 vol% | 1.50 | 3.20 | 5.70 |
| | | 0.2 vol% | 1.65 | 3.20 | 5.50 |
| Diluted to 5 times | Light conditions 1 | 0 vol% | 3.75 | 6.00 | 5.50 |
| | | 0.05 vol% | 4.65 | 6.70 | 7.20 |
| | | 0.1 vol% | 4.65 | 6.80 | 6.50 |
| | | 0.2 vol% | 4.20 | 6.50 | 6.20 |
| | Light conditions 2 | 0 vol% | 3.05 | 4.30 | 4.60 |
| | | 0.05 vol% | 3.40 | 5.60 | 4.90 |
| | | 0.1 vol% | 5.90 | 6.90 | 6.10 |
| | | 0.2 vol% | 7.10 | 6.80 | 6.10 |

As seen from Table 3 and Table 4, the growth of microalgae at the initial stage of the cultivation was faster in the cultivation in the liquid in which Beer 1 or Beer 2 was diluted to 5 times. Further, in the cultivation in the liquid in which Beer 1 or Beer 2 was diluted to twice, microalgae can grow on the 5th to 7th day.

When the liquid further contains yeast autolysate, the growth of microalgae is more enhanced.

Further, the growth of microalgae is better under the light conditions 1 than under the light conditions 2.

**Table 5**

| (Test example 3) | | | | | |
|---|---|---|---|---|---|
| Unit: Dry weight g/L | | | | | |
| Sake | | Concentration of yeast autolysate | Day 3 | Day 5 | Day 7 |
| Diluted to 5 times | Light conditions 1 | 0 vol% | 2.40 | 3.90 | 4.50 |
| | | 0.05 vol% | 3.30 | 5.90 | 7.90 |
| | | 0.1 vol% | 4.00 | 7.90 | 11.20 |
| | | 0.2 vol% | 3.80 | 7.30 | 10.90 |
| | Light conditions 2 | 0 vol% | 1.13 | 2.80 | - |
| | | 0.05 vol% | 1.30 | 3.90 | - |
| | | 0.1 vol% | 1.20 | 4.20 | - |
| | | 0.2 vol% | 1.33 | 4.00 | - |
| Diluted to 15 times | Light conditions 1 | 0 vol% | 2.30 | 3.60 | 3.50 |
| | | 0.05 vol% | 3.80 | 5.10 | 3.30 |
| | | 0.1 vol% | 4.40 | 5.40 | 3.80 |
| | | 0.2 vol% | 3.90 | 5.30 | 3.90 |
| | Light conditions 2 | 0 vol% | 1.03 | 2.70 | - |
| | | 0.05 vol% | 1.03 | 3.80 | - |
| | | 0.1 vol% | 1.30 | 3.90 | - |
| | | 0.2 vol% | 1.60 | 3.70 | - |

As seen from Table 5, when the liquid further contains yeast autolysate, the growth of microalgae is more enhanced.

Further, the growth of microalgae is better under the light conditions 1 than under the light conditions 2.

On the other hand, microalgae were cultured under the light conditions 1 and the light conditions 2 in the same manner, using a liquid containing 2.5% of ethanol (containing ethanol equivalent to a solution in which Beer 1 was diluted to twice) and a liquid containing 1% of ethanol (containing ethanol equivalent to a solution in which Beer 1 was diluted to 5 times), instead of the liquid containing Beer 1 of Test example 1 (Test example 4).

In Test example 4, the amount of components derived from the AF-6 composition in the liquid A was the same. However, ammonium sulfate and potassium dihydrogen phosphate (KH₂PO₄) were mixed in the liquid as inorganic nutrients (as a phosphorus source and a nitrogen source). The contents of ammonium sulfate and potassium dihydrogen phosphate in the liquid were adjusted so that the total nitrogen concentration and the total phosphorus concentration should be the same as those in Test example 1 that used Beer 1. Specifically, the concentrations of ammonium sulfate and potassium dihydrogen phosphate in the liquid A were adjusted, as shown in Table 6, so that T-N (total nitrogen content) and TP (total phosphorus content) were the same as those in Test example 1.

**Table 6**

| | Ethanol | Ammonium sulfate | KH₂PO₄ |
|---|---|---|---|
| Equivalent to solution of Test example 1 in which beer was diluted to twice | 2.5% | 1079.6 mg/L | 458.5 mg/L |
| Equivalent to solution of Test example 1 in which beer was diluted to 5 times | 1.0% | 431.8 mg/L | 183.4 mg/L |

**Table 7**

| (Test example 4) | | | | | |
|---|---|---|---|---|---|
| Unit: Dry weight g/L | | | | | |
| Ethanol | | Concentration of yeast autolysate | Day 3 | Day 5 | Day 7 |
| 2.5% concentration | Light conditions 1 | 0 vol% | 1.90 | 2.70 | 3.90 |
| | | 0.05 vol% | 2.40 | 3.50 | 5.30 |
| | | 0.1 vol% | 2.20 | 3.60 | 4.90 |
| | | 0.2 vol% | 2.50 | 4.30 | 5.50 |
| | Light conditions 2 | 0 vol% | 1.65 | 2.10 | 2.60 |
| | | 0.05 vol% | 1.70 | 3.30 | 3.90 |
| | | 0.1 vol% | 1.30 | 1.20 | 2.10 |
| | | 0.2 vol% | 1.30 | 1.50 | 2.20 |
| 1.0% concentration | Light conditions 1 | 0 vol% | 2.75 | 4.10 | 3.80 |
| | | 0.05 vol% | 2.50 | 4.40 | 3.70 |
| | | 0.1 vol% | 2.85 | 4.60 | 3.70 |
| | | 0.2 vol% | 3.30 | 5.40 | 3.90 |
| | Light conditions 2 | 0 vol% | 2.30 | 3.10 | 3.20 |
| | | 0.05 vol% | 2.75 | 3.80 | 3.60 |
| | | 0.1 vol% | 3.20 | 4.10 | 3.90 |
| | | 0.2 vol% | 3.90 | 4.20 | 3.80 |

As seen from Test examples 1 to 3 and Test example 4, when microalgae are cultured in a liquid containing a brewed beverage, the microalgae can be efficiently grown.

Further, use of a brewed beverage as a material containing organic nutrients together with aeration (aeration by shaking) and further photosynthesis of the microalgae enhances the culture efficiency more than sole use of ethanol as organic nutrients.

Further, it can be seen that an excellent biomass production rate can be ensured by using a brewed beverage.

### INDUSTRIAL APPLICABILITY

The method for culturing microalgae of the present invention can be used suitably for using microalgae having organic compounds such as hydrocarbons and polysaccharides stored within their cells for applications such as health foods, drugs, feeds, chemical products, and fuels. Specifically, the method for culturing microalgae of the present invention can be used suitably for using microalgae as a raw material of fuels, for example, by employing the organisms belonging to the genus *Euglena* as the microalgae, culturing the microalgae so as to allow the microalgae to store oils within their cells, and extracting the oils.

### REFERENCE SIGNS LIST

1: Culture Tank
2: Preparation Tank
3: Sterilizing Tank
4: Concentrating Device
5: Dehydrating Device
A: Liquid
B: Light

## Claims

1. A method for culturing microalgae in a liquid at least containing a brewed beverage.

2. The method for culturing microalgae according to claim 1, wherein
the microalgae are cultured in a liquid containing the brewed beverage and at least one selected from the group consisting of sweet wort, sake lees, shochu lees, yeast, yeast autolysate, and blackstrap molasses.

3. The method for culturing microalgae according to claim 1 or 2, wherein
the brewed beverage is at least one selected from the group consisting of beer, sake, and wine.

4. The method for culturing microalgae according to any one of claims 1 to 3, wherein
the microalgae are cultured in the liquid containing oxygen while performing photosynthesis by irradiation with light.

5. The method for culturing microalgae according to any one of claims 1 to 4, wherein
the microalgae are cultured in facilities for producing beer as the brewed beverage and in a liquid containing the beer generated in the facilities.

6. The method for culturing microalgae according to any one of claims 1 to 5, wherein
the microalgae are cultured in the facilities for producing the beer as the brewed beverage and in a liquid containing the beer generated in the facilities and at least one of the sweet wort, the yeast, and the yeast autolysate that are generated in the facilities.

7. The method for culturing microalgae according to any one of claims 1 to 4, wherein
the microalgae are cultured in facilities for producing sake as the brewed beverage and in a liquid containing the sake generated in the facilities.

8. The method for culturing microalgae according to any one of claims 1 to 5, wherein
the microalgae are cultured in facilities for producing sake as the brewed beverage and in a liquid containing the sake generated in the facilities and at least one of the sake lees, the yeast, and the yeast autolysate that are generated in the facilities.

9. A method for using microalgae obtained by the method for culturing microalgae according to any one of claims 1 to 8 at least as fuels in the facilities in which the culture method has been implemented.
